# EUROPEAN PATENT SPECIFICATION

(11) **EP 1 085 924 B1**
(45) Date of publication and mention of the grant of the patent: **26.04.2006**
(21) Application number: 98926067.4
(22) Date of filing: 26.05.1998
(51) Int. Cl.: A61H 33/14, A61G 10/02, A61M 37/00

(54) **SHAPE-ADAPTABLE TOPICAL HYPERBARIC OXYGEN CHAMBER**
FORMANPASSBARE HYPERBARISCHE TOPISCHE SAUERSTOFFDRUCKKAMMER
CHAMBRE A OXYGENE HYPERBARE, TOPIQUE ET DE TAILLE REGLABLE

(43) Date of publication of application: 28.03.2001
(73) Proprietor: Numotech Incorporated, Sun Valley, CA 91352 (US)
(72) Inventor: HENG, Madalene, C., Y., Northridge, CA 91325 (US)
(74) Representative: Ruschke, Hans Edvard
(86) International application number: PCT/US1998/010670
(87) International publication number: WO 1999/061094

(56) References cited:
- WO-A-98/17340
- US-A- 3 450 450
- US-A- 3 754 731
- US-A- 4 003 371
- US-A- 5 312 385
- US-A- 5 342 121
- US-A- 5 478 310
- US-A- 5 542 414

## Description

### BACKGROUND OF THE INVENTION

### 1) FIELD OF THE INVENTION

The present invention relates to a hyperbaric chamber and more particularly to a hyperbaric chamber for topically applied gas which includes oxygen which is portable and disposable and is designed to be used in connection with the torso and two or more limbs of an animal, such as a human body, creating a sealed environment for the application of oxygen containing gas to hasten healing of wounds such as skin ulcers, lesions and injuries on a patient's body.

### 2) DESCRIPTION OF THE PRIOR ART

Hyperbaric oxygen chambers have long been known. Hyperbaric oxygen chambers are for the purpose of introducing of pressurized oxygen into an encapsulated environment with this oxygen to promote the healing of various types of wounds. Specifically, it has been discovered that the treatment of wounds within an hyperbaric oxygen chamber promotes healing and suppresses bacterial infection.

When hyperbaric oxygen chambers were first introduced, such took the form of a rigid, heavy structure resembling an iron lung. The entire body of the patient, up to the neck of the patient, was placed within the chamber. One of the disadvantages of this type of hyperbaric oxygen chamber is that the patient had to be moved to the chamber. Also, because of the size and weight of the hyperbaric oxygen chamber, this type of hyperbaric oxygen chamber was quite expensive and therefore a typical hospital may only have one or two such chambers making it difficult to find the time to use the chamber(s) in conjunction with many different patients. Additionally, because of the possibility of transferred infection from one patient to another, time and effort had to be expended to clean the chamber between uses. All in all, such "iron lung" type of hyperbaric oxygen chambers were not convenient.

As time progressed, hyperbaric oxygen chambers became more sophisticated resulting in the production of a portable hyperbaric oxygen chamber. Not only was the hyperbaric oxygen chamber portable, it was inexpensive permitting the hyperbaric oxygen chamber to be disposed of after usage. These more sophisticated hyperbaric oxygen chambers came in various sizes some of which could basically encapsulate the entire torso of the patient or smaller versions of the chamber which would be used to encapsulate only small portions of the torso, such as an arm or a leg. An example of such a prior art arrangement is shown in WO 98/17340 on which the pre-characterizing part of claim 1 is based. However, the portable hyperbaric oxygen chambers of the prior art had certain shortcomings. One of the shortcomings is that it is important that the portable hyperbaric oxygen chamber assume a very loose fit around the patient's body so that a substantial volume of oxygen will be subjected to the wound. A close fit of the chamber to the body is undesirable. Many of the portable hyperbaric oxygen chambers of the prior art failed to adequately provide this type of loose fit.

Another shortcoming had to do with the connection of the gas supply tube to the chamber which may consist of a plastic bag. Frequently, the gas supply tube would have a tendency to kink right at the connection with the portable chamber which most often comprises a flexible thin-walled plastic bag. This kinking would result in diminishing or completely shutting off of the supply of gas into the internal chamber of the bag. In order for the hyperbaric chamber to work satisfactorily, a constant continuous supply of gas is required.

Another shortcoming of the prior art hyperbaric oxygen chambers is that at times a patient may be located in the chamber for a period of several hours, and this patient may even be unconscious. The patient may urinate. The prior art types of hyperbaric oxygen chambers were not constructed in a manner to facilitate the removal of the urine.

Another shortcoming of the prior art hyperbaric chambers is that there was no way to perform any kind of manual procedure on the patient through the wall of the chamber (bag). For example, sometimes it is necessary to move the patient or move a particular portion of the patient's anatomy from one location to another. Also, at times it is desirable to do some massaging to a particular area of the patient's body. Satisfactory contact with the patient's body is usually not possible with the prior art type of portable hyperbaric oxygen chambers because the wall thickness of the chamber is too great to facilitate manual procedures.

Another shortcoming is the lack of maintenance of the intrachamber pressures at a narrow range necessary for healing of skin ulcers, lesions and injuries. In order to maximize the growth of new blood vessels within the wound and retard the excessive formation of scar tissue, the pressures within the hyperbaric oxygen chamber must be maintained at all times within a narrow window of pressures within the chamber. Such a narrow "window" of pressure maintenance is not known in the prior art.

### SUMMARY OF THE INVENTION

The present invention is defined in the appended claim 1 overcomes the deficiency of the prior art hyperbaric oxygen chambers and provides such a chamber for treating wounds which include skin ulcers, injuries and lesions. This chamber is disposable, portable, and inexpensive. In one embodiment the structure eliminates the possibility of kinking the bag at the gas supply tube that is connected to the chamber. The structure may also permit the draining of any accumulated liquid within the chamber, and may provide a way to perform physical operations on the patient's body while still maintaining a pressurized environment within the topical hyperbaric oxygen chamber. Most importantly, the hyperbaric oxygen chamber of this invention provides a way to ensure that the intrabag pressures are maintained at all times within the narrow "window" necessary to maximize the growth of new blood vessels while depressing the formation of excessive scar tissue. There is no known treatment to date that is capable of dissociating growth of scar tissue from growth of new blood vessels. The hyperbaric oxygen chamber of this invention utilizes an enclosing bag which is to have a single access opening. Substantially the entire torso (trunk) of the animal, which in most cases would be a human, is to be inserted within the internal chamber of the bag with the wall of the bag assuming a very loose fit in conjunction with the body of the human. Around the access opening of the bag, the wall of the bag is pleated to permit localized expansion of the bag in order to increase the volume of the internal chamber in the area directly over the wound. In the described embodiment the wall of the bag has at least one supply connector which is connected to the gas source which includes oxygen. This supply connector has an enlarged flange and a tubular stem. This tubular stem is for connection with the oxygen supply tube. The enlarged flange is secured to the wall of the bag. This enlarged flange prevents pivoting of the connector relative to the bag which could result in kinking of the gas supply and the decreasing or not supplying of gas to the internal chamber of the bag. Also included within the wall of the bag is a drain connector, the function of which is to connect with a drain tube for removal of accumulated liquid within the internal chamber. Also formed within the wall of the bag will be at least one access opening for manipulating the patient while under treatment. Within this access/opening is a thin-walled glove. This glove is readily flexible and is reversible by insertion of the hand of the caregiver into the glove. This will permit the caregiver to manipulate the patient within the sealed chamber.

One of the objectives of the present invention is to provide a hyperbaric oxygen chamber wherein the chamber itself is collapsible and can be stored in a substantially small amount of space prior to usage.

Another objective of the present invention is to construct a hyperbaric chamber which is inexpensive and disposable.

Another objective of the present invention is to provide a hyperbaric oxygen chamber which is light in weight so that it can be readily moved from a stowed position to the location of the patient rather than vice versa, and also the light weight achieves the advantage of minimal cost of shipping of the hyperbaric oxygen chamber to the medical facility.

Another objective of the present invention is to construct a hyperbaric oxygen chamber wherein the patient can move comfortably within the chamber and is not required to maintain a virtually rigid position for the time of treatment.

Another objective of the present invention is to construct a hyperbaric oxygen chamber which is basically transparent so that the body of the human can be readily viewed during the time of treatment.

Another objective of the present invention is that by constructing a hyperbaric chamber for topically applied gas which includes oxygen and which is disposable, there is eliminated the need for any cleaning of such chambers and also eliminates the transfer of infection which is a possibility when using such a chamber on multiple patients.

Another objective of the present invention allows for the incorporation of pressure sensors in conjunction with the chamber in order to maintain a narrow window of pressures that is necessary for maximizing growth of new blood vessels while at the same time suppressing the formation of scar tissue.

Another objective of the present invention is to construct the bag of the hyperbaric oxygen chamber to be readily changed in shape in order to accommodate and supply a substantial volume of gas to difficult locations of wounds on the body of the patient.

### BRIEF DESCRIPTION OF THE DRAWINGS

Figure 1 is a top plan view of the hyperbaric oxygen chamber constructed in accordance with this invention showing such chamber (enclosing) being mounted on a typical human patient;
Figure 2 is a top plan view of a portion of the hyperbaric oxygen chamber constructed in accordance with this invention wherein the chamber includes a plurality of hand access openings showing reversible tubular glove members of the hand access openings in an exterior protruding position from the chamber;
Figure 3 is a view similar to Figure 2 but showing the tubular glove members in the reversed position within the interior of the chamber;
Figure 4 is an enlarged view of the body access opening of the thin-walled bag of the hyperbaric oxygen chamber showing more clearly the mounting and sealing of the bag onto the body of the patient with this view being taken along line 4-4 of Figure 1;
Figure 5 is a cross-sectional view through the pleated area of the bag located directly adjacent the body access opening taken along line 5-5 of Figure 4;
Figure 6 is an enlarged cross-sectional view through the first embodiment of gas connector which is mounted in conjunction with the wall of the bag taken along line 6-6 of Figure 1;
Figure 7 is a cross sectional view of a second embodiment of gas connector which is mounted in conjunction with the wall of the bag;
Figure 8 is a side elevational view of a Bourdon tube type of pressure sensor that can be utilized in conjunction with a hyperbaric oxygen chamber of the present invention showing the pressure sensor in the position of detecting only a small increase in pressure above ambient pressure;
Figure 9 is a side elevational view of the pressure sensor of Figure 8 showing the pressure sensor in the position of detecting an excessive pressure level;
Figure 10 is a side elevational view of the pressure sensor that is mounted in conjunction with the hyperbaric oxygen chamber of the present invention showing the pressure sensor in its correct position detecting the correct amount of pressure within the hyperbaric oxygen chamber; and
Figure 11 is a cross-pectional view through the sensor taken along line 11-11 of Figure 9.

### DETAILED DESCRIPTION OF THE PREFERRED EMBODIMENT

Referring particularly to the drawings, there is depicted a human 10 positioned in a prone position on a bed 12. The human 10 has a torso or trunk 14. A portable hyperbaric oxygen chamber 16 of the present invention is mounted in conjunction with the human 10 in the following manner.

The chamber 16 is primarily composed of an enlarged, sheet material plastic bag 18. A typical material of the bag 18 would be polyethylene. The bag 18 has a single access opening 20 which provides access into the internal chamber 22 of the bag 18. The access opening 20 is to be large enough that the legs 24 and 26 of the human 10 are to be locatable within the internal chamber 22 with the access opening 20 being positioned about the torso 14. It is important that the bag 18 be transparent.

The typical thickness of the bag 18 will generally be about 0.15 mm (0.006 of an inch). The bag 18 directly adjacent to the access opening 20 includes a plurality of pleats 28. The pleats 28 are formed by a series of parallel, spaced apart score lines 30, with sections of the polyethylene folded upon itself, each fold being defined as a pleat. These pleats 28 facilitate the bunching up, overlapping or folding over of the pleats on each other so as to accommodate the different circumferences of the torso 14. Not only do the pleats 28 facilitate the adjustment to different sizes of torso 14, but also this overlapping of the pleats establishes a substantially close fit with the torso 14. The bag 18 in the area of the access opening 20 is filled around the torso 14 by means of a sealing tape 32. The sealing tape 32 is to be placed about the torso 14 and also about the access opening 20 of the bag 18 with the ends of the sealing tape 32 being secured to each other also in an overlapping arrangement. It is to be understood that the sealing tape 32 will include some kind of adhesive. As previously mentioned, there will be a certain amount of leakage of the pressurized gas mixture which includes oxygen, that is to be supplied within the internal chamber 22. This leakage will occur in the area of the sealing tape 32. It is desirable to have a small amount of leakage so that the gas contained within the internal chamber is constantly being changed.

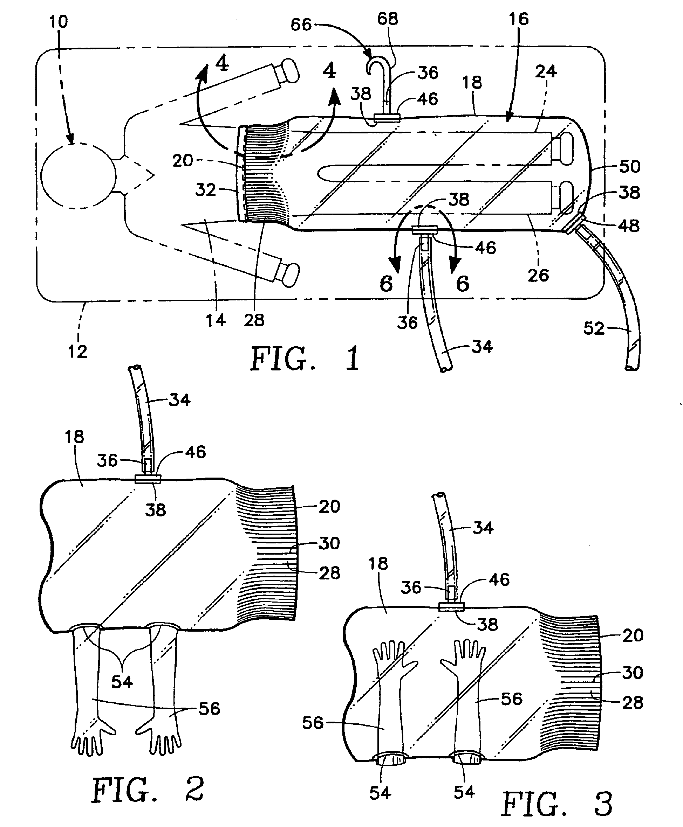

Gas which includes oxygen is to be supplied from a source (not shown) through a gas supply tube 34 to within the internal chamber 22. The gas supply tube 34 is to be tightly mounted onto an upstanding connector stem 36 of a first embodiment of connector shown in Figures 1-3 and 6. The connector stem 36 is integral with an enlarged flange 38 with the flange being disc shaped and extending in a right angle outwardly from the stem 36. The connector stem 36 has a longitudinal through opening 40 and also a serrated exterior surface 42. The serrated exterior surface 42 will facilitate a snug fit with the gas supply tube 34. Also, the serrated exterior surface 42 will tend to resist disengagement of the gas supply tube 34 from the connector stem 36.

The connector stem 36 is to be inserted through a hole 44 formed within the bag 18. The flange 38 is to be located directly adjacent the interior surface of the bag 18. Mounted against the exterior surface of the bag 18 surrounding the connector stem 36 is a washer 46. Both the washer 46 and the flange 38 are to be tightly sealed to the bag 18. A typical method of sealing would be by means of heat.

It is the function of the connector stem 36, in conjunction with the flange 38 and the washer 46, to establish a non-kinking relationship between the bag 18 and the gas supply tube 34. Regardless of the position of the gas supply tube 34, there is to be always an open gas flow path between the gas supply tube 34 and the through opening 40, therefore, gas will always be free to flow from the gas supply tube 34 into the internal chamber 22.

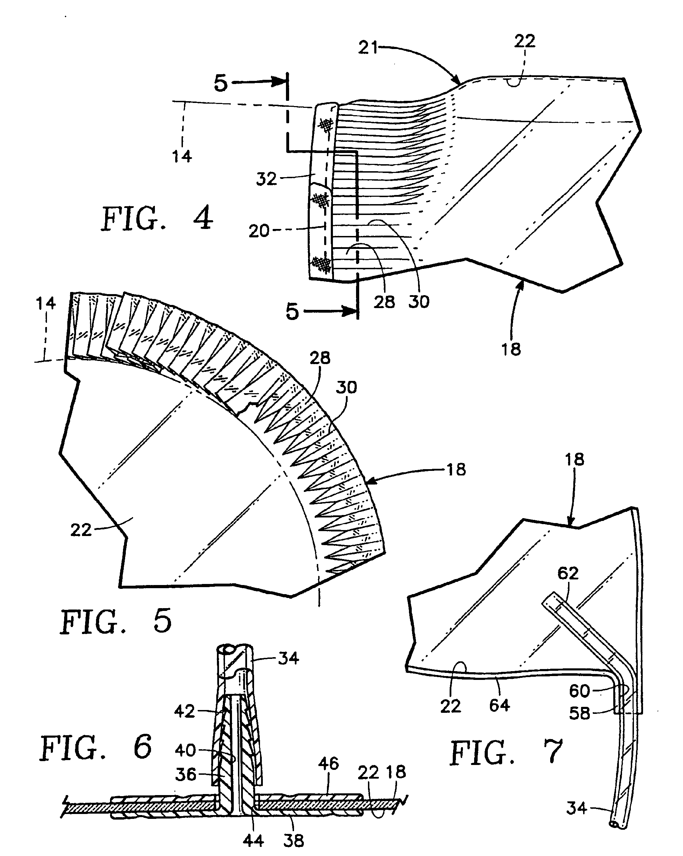

Also mounted in conjunction with the bag 18 is a drain connector 48. The drain connector 48 is constructed in precisely the same way as the connector that is shown in Figure 6 of the drawings. The drain connector 48 is mounted at the foot area 50 of the bag 18. Also, the drain connector 48 is to be located directly adjacent the bed 12. It is to be the function of the drain connector 48 to discharge any accumulated liquid within the internal chamber 22 into a drain hose 52. The liquid is to be discharged to an appropriate exterior discharge location which is not shown.

Formed within the wall surface of the bag 18 are a pair of hand access openings 54. Mounted in conjunction with each hand access opening 54 is a thin-walled, tubular member 56 which is constructed of rubber or plastic. The tubular member 56 is elongated and terminates at its outer end in the shape of a glove for a human hand. Each tubular member 56 establishes an airtight connection with the its respective hand access opening 54. One tubular member 56 is designed to accommodate the medical practitioner's left hand with the other tubular member 56 being designed to accommodate the medical practitioner's right hand.

Normally with gas being supplied within the internal chamber 22 being under pressure, the tubular members 56 will be positioned in an outwardly protruding position from the bag 18 as shown in Figure 2. However, upon a medical practitioner desiring to perform certain operations on the human patient 10, such as manipulating an area of the human patient's body, the medical practitioner is able to forcibly move each of the tubular members 56 to within the internal chamber 22 by inserting each of the caregiver's hands in conjunction with a tubular member 56. This will locate the caregiver's hands within the internal chamber 22 permitting the caregiver to perform the desired physical function on the human patient 10. The typical material of construction for the tubular members 56 will be thin, surgical rubber which is commonly used in the making of surgical type of gloves used in medical operations.

When the caregiver has completed performing whatever physical operation is desired on the human patient 10, the medical practitioner is to merely withdraw his or her hands and arms from the internal chamber 22 through the respective access openings 54 which will result in the tubular members 56 again assuming the outwardly protruding position shown in Figure 2. The usage position in conjunction with the human patient is shown in Figure 3 of the drawings.

It is possible that the sealing tape 32 could be adjusted so tightly that very little oxygen is permitted to escape. In such a case, the sealing tape 32 would be loosened, somewhat, so that the pressure of the gas within the internal chamber 22 is maintained within a narrow window of pressure required for wound heating. Typically, the bag 18 is to be manufactured to be 1.83 m to 2.44 m (six to eight feet) long and 0.91 m to 1.22 m (three to four feet) wide.

It is to be restated that one of the principal features of utilizing the chamber 16 of the present invention is that when it is observed that the bag 18 is closely located against a wound the medical practitioner only needs to pull the bag 18 in an outward direction adjacent the wound creating a bulge 21. Even if this wound is located directly adjacent the sealing tape 32, bunching of the pleats 28 over the area of the wound will permit a localized expansion of the bag 18 into bulge 21. This expansion of the bag 18 into bulge 21 in the desired area is for the purpose of increasing of the volume of oxygen that comes into contact with the wound necessary to enhance healing.

Referring particularly to Figure 7 of the drawings, there is shown a second embodiment of non-kinking connection of the oxygen supply tube 34 to the bag 18. At a corner of the bag 18, there is formed a right angled extension 58. This right angled extension 58 includes a hole 60 that closely conforms in size to the oxygen supply tube 34. The gas supply tube 34 is to be pushed through the hole 60 until the inner end 62 of the gas supply tube 34 extends about 203 mm (eight inches) within the internal chamber 22 of the bag 18. The bag 18 must include seams at various locations. Forming of the extension 58 is to be at one of those seams 64. The locating of the gas supply tube 34 at the seam 64 further reduces the possibility of any kinking occurring. Once the gas supply tube 34 is installed in position with the inner end 62 extending about 203 mm (eight inches) within the internal chamber 22, an appropriate sealing is to occur between the extension 58 and the oxygen supply tube 34. This type of sealing could either be by adhesive, heat sealing or even an exterior tape.

An important feature of this invention is that if the pressure level of the gas within the bag 18 is not maintained to within a certain narrow "window" at a selected pressure, the hyperbaric oxygen chamber of this invention will not be nearly as effective in healing of wounds. For all known wounds the desired range of pressure should be 67 Pa-1.33 kPa (0.5 to 10 millimeters of mercury (mm of Hg)) above ambient pressure. This narrow window of pressure above ambient pressure is necessary to both exclude reperfusion injury and prevent oxygen toxicity. Reperfusion injury is injury which is caused by the pressure being below this narrow window pressure range, and oxygen toxicity is what occurs when the pressure level is above the range of the narrow window pressure. Within this precise range, the biological process is altered such that the development of scar tissue is retarded while at the same time blood vessel growth is accelerated. The net result of this is that less scar tissue is generated and more vascular dense, healthy tissue is laid down in the wound. These healed wounds can withstand considerably more pressure than scar tissue and consequently these healed wounds do not tend to rebreak down. Therefore, a simple but effective way must be used to make it readily apparent to the caregiver, which is usually a doctor or a nurse, that the correct amount of pressure is being maintained within the bag 18.

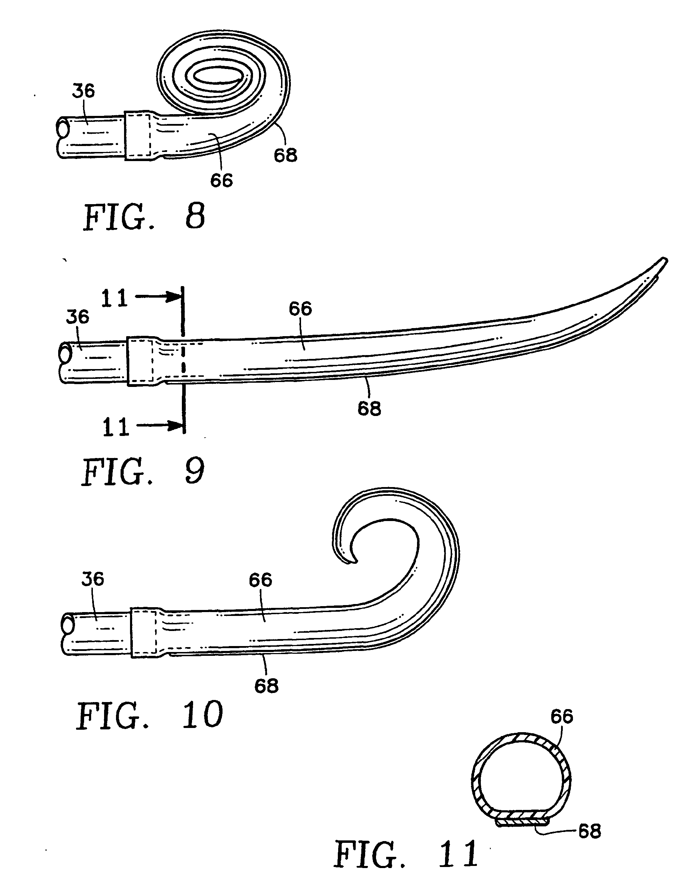

Types of sensors that may be utilized in conjunction with the bag 18 are manometric, liquid crystal, silicon chip and Bourdon tube. Referring particularly to Figures 1, 8, 9, 10 and 11 of the drawings, there is shown a Bourdon tube type of pressure sensor incorporated with the bag 18. The Bourdon tube type comprises a spiral plastic tube 66 which is adhered on one side to a thin metal strip 68. The thin metal strip 68, when at rest, has a tendency to coil into a spiral. The single entry opening into the plastic tube 66 is mounted onto a connector stem 36 which is constructed in accordance with the structure shown in Figure 6 of the drawings. The pressure of the gds within the bag 18 is supplied within the plastic tube 66. If the pressure is too low, the sensor will be in the position as shown in Figure 8. If the pressure is too high, the sensor will be in the position as shown in Figure 9. The correct position for the sensor is shown in Figures 1 and 10. By the caregiver making a momentary glance of the sensor, the caregiver can quickly determine if the pressure level within the bag 18 is too low, too high, or just right. It is to be understood that normally there will be incorporated a pressure regulator (not shown) on the gas supply source. It is important that whatever sensor is utilized in conjunction with the bag 18 that it be as inexpensive as possible so that the bag 18 can be manufactured to be disposable. With this thought in mind, this is why the Bourdon tube type of sensor shown in Figures 1,8-11 of the drawings would be desirable because it is inherently inexpensive. Also, a silicon chip type of sensor may also be sufficiently inexpensive.

## Claims

1. A portable disposable topical hyperbaric oxygen chamber (16) for localised treatment of wounds on a portion (14, 22, 26) of a human or animal body (10) comprising:
an enclosing bag (18) having an internal chamber (22), said enclosing bag (18) being adapted in use to receive a gas including oxygen pressurised within the range of 66.7 Pa to 1.33 KPa (0.5 to 10 mmHg) above ambient pressure, said enclosing bag (18) having a single body access opening (20) which in use allows entry into said internal chamber (22) of said portion (14, 22, 26) of the human or animal body with the portion (22, 26) of the body being conducted through said body access opening (20) and located within said internal chamber (22), said enclosing bag (18) having a wall constructed of thin, flexible, transparent material; and
sealing means (32) applied to said wall at said body access opening (20) adapted for forming a substantially airtight seal between said enclosing bag (18) and the portion of the body (14, 22, 26) permitting only slow leakage of said gas from said internal chamber (22);
**characterized in that**
said wall of said bag (18) around said body access opening (20) including a series of preformed pleats (28), said access opening (20) having an initial opening size, said preformed pleats (28) being formed by a series of parallel spaced apart score lines (30) formed within said wall, said preformed pleats (28) permitting expanding of said initial opening size of said access opening (20) as well as permitting the contraction of said initial opening size of said access opening (20), said preformed pleats (28) facilitating gathering of said wall at said access opening to form a close fit with the portion (14, 22, 26) of the human or animal body, said preformed pleats (28) when bunched together allowing localised shape changes of the bag to increase the volume of oxygen over the area of the wound.

2. A chamber according to claim 1, wherein said wall has at lease one drain connector (48), said drain collector (48) adapted to connect with a drain tube (52) for removal of accumulated liquid from within said internal chamber (22).

3. A chamber according to claim 1 or 2, wherein at least one hand access opening (54) formed within said wall, said hand access opening (54) including a tubular glove member (56), said tubular glove member (56) being formed of a thin, flexible, airtight material, said tubular glove member (56) being movable between an exterior position and an interior position, said exterior position locating said tubular glove member exteriorly of said wall, said interior position locating said tubular glove member (56) within said internal chamber (22), whereby a person's hand is to connect with said tubular glove member (56) and move said tubular glove member (56) within said internal chamber (22) permitting the person's hand to perform functions on said portion of the body (14, 24, 26) within said internal chamber (22) while maintaining a gas pressurized environment of said gas within said internal chamber (22).

4. The chamber as defined in any preceding claim wherein:
said wall having at least one supply connector (36, 38, 46) adapted to connect with a gas supply tube (34) to supply said gas, said supply connector (36, 38, 46) having a male tubular stem (36) mounted on an enlarged flange (46, 38), said enlarged flange being fixedly secured to said wall, whereby said enlarged flange (46, 38) prevents kinking of said gas supply tube (34) ensuring continuous supply of gas through the gas supply tube (34) into said internal chamber (22).

5. The chamber as defined in any preceding claim, wherein:
a pressure sensor (66) being connected to said enclosing bag (18), said pressure sensor (66) being capable of denoting the pressure level of said gas in the enclosing bag (18) to an observer during operation of said hyperbaric oxygen chamber 816).

6. The chamber as defined in claim 1, wherein there is provided a gas supply tube (34) and the bag (18) has a right-angled extension (58) at one corner of the bag (18), the gas supply tube (34) extending through a hole (60) in the right-angled extension (58) to a distance of about 203 mm (8 inches) within the internal chamber (22) of the bag (18) thereby reducing the possibility of kinking of the gas supply tube (34).

## Patentansprüche

1. Transportable topische hyperbare Einweg-Sauerstoffkammer (16) für die lokalisierte Behandlung von Wunden am Teil (14, 22, 26) eines Menschen- oder Tierkörpers (10) mit:
einem einhüllenden Sack (18) mit einer inneren Kammer (22), der im Einsatz ein Gas einschl. Sauerstoff aufnehmen kann, das mit einem überatmosphärischen Druck im Bereich von 66,7 Pa bis 1,33 kPa (0.5 bis 10 mm Hg) beaufschlagt ist, wobei der einhüllende Sack (18) eine einzige Körperzugangsöffnung (20) enthält, durch die im Einsatz der Teil (14, 22, 26) des Menschen- oder Tierkörpers in die innere Kammer (22) einführbar ist, wobei weiterhin der Teil (22, 26) des Körpers durch die Körperzugangsöffnung (20) hindurch in die innere Kammer (22) eingebracht wird und wobei schließlich der einhüllende Sack (18) eine Wand aus einem dünnen flexiblen transparenten Material aufweist; und
einer Verschluss- und Abdichteinrichtung (32), die an der Körperzugangsöffnung (20) auf die Wand aufgebracht ist und mit der sich zwischen dem Sack (18) und dem Körperteil (14, 22, 26) ein im wesentlichen luftdichter Abschluss herstellen lässt, der das Gas aus der inneren Kammer (22) nur langsam entweichen lässt,
**dadurch gekennzeichnet, dass**
die Wand des Sacks (18) eine anfängliche Öffnungsgröße hat und um die Körperzugangsöffnung (20) herum von einer Folge vorgeformter Falten (28) umgeben ist, die von einer Folge in der Wand ausgebildeter paralleler beabstandeter Schwächungslinien (30) gebildet sind und ein Vergrößern sowie ein Kontrahieren der anfänglichen Öffnungsgröße der Zugangsöffnung (20) ermöglichen, wobei die vorgeformten Falten ein Raffen der Wand an der genannten Zugangsöffnung zwecks Ausbildung eines dichten Sitzes auf dem Teil (14, 22, 26) des Menschen- oder Tierkörpers erleichtert und im gerafften Zustand lokalisierte Formänderungen des Sacks erlaubt, um das Sauerstoffvolumen über dem Wundbereich zu vergrößern.

2. Kammer nach Anspruch 1, bei der die Wand einen Ablassanschluss (48) aufweist, an den ein Ablassschlauch (52) zum Beseitigen von angesammelter Flüssigkeit aus der inneren Kammer (22) anschließbar ist.

3. Kammer nach Anspruch 1 oder 2, bei der in der Wand mindestens eine Handeinführöffnung (54) mit einem rohrförmigen Handschuhelement (56) aus einem dünnen flexiblen luftdichten Material ausgebildet ist, wobei das Handschuhelement (56) zwischen einer Außen- und eine Innenposition bewegbar ist und sich in der Außenposition außerhalb der Wand und in der Innenposition innerhalb der Kammer (22) befindet und wobei die Hand einer Person in das Handschuhelement (56) einführbar und in der inneren Kammer (22) bewegbar ist, so dass die Hand auf dem in der Kammer (22) befindlichen Körperteil (14, 24, 26) Funktionen ausführen kann, während in der Kammer (22) das druckbeaufschlagte Gasmilieu erhalten bleibt.

4. Kammer nach einem der voranstehenden Ansprüche, bei der die Wand mindestens einen Speiseanschluss (36, 38, 46) enthält, an den eine Gasspeiseleitung (34) zur Zufuhr des Gases anschließbar ist, wobei der Anschluss (36, 38, 46) einen steckbaren rohrförmigen Schaft (36) aufweist, der auf einen vergrößerten, mit der Wand fest verbundenen Flansch (46, 38) aufgesetzt ist, und wobei der Flansch (46, 38) ein Abknicken der Gasspeiseleitung (34) verhindert und so eine stetige Gaszufuhr durch die Gasspeiseleitung (34) in die innere Kammer (22) sicherstellt.

5. Kammer nach einem der voranstehenden Ansprüche, bei der an den einhüllenden Sack (18) ein Druckfühler (66) angeschlossen ist, mit dem im Einsatz der hyperbaren Sauerstoffkammer (16) einem Beobachter der Gasdruck im Sack (18) anzeigbar ist.

6. Kammer nach Anspruch 1, bei der eine Gasspeiseleitung (34) vorgesehen ist und der Sack (18) an einer Ecke eine rechtwinklige Verlängerung (58) hat, wobei die Gasspeiseleitung (34) durch ein Loch (60) in der rechtwinkligen Verlängerung (58) etwa 203 mm (8 in.) weit in die innere Kammer (22) des Sacks (18) vorsteht, um die Möglichkeit eines Abknickens der Gasspeiseleitung (34) zu verringern.

## Revendications

1. Chambre à oxygène hyperbare topique jetable et portable (16) pour le traitement localisé de plaies sur une partie (14, 22, 26) d'un corps humain ou animal (10) comprenant :
un sac enveloppant (18) présentant une chambre interne (22), ledit sac enveloppant (18) étant adapté à l'utilisation pour recevoir un gaz incluant de l'oxygène pressurisé dans l'intervalle de 66,7 Pa à 1,33 KPa (0,5 à 10 mmHg) au-dessus de la pression ambiante, ledit sac enveloppant (18) présentant une seule ouverture d'accès au corps (20) qui, à l'utilisation, permet de pénétrer à l'intérieur de ladite chambre interne (22) de ladite partie (14, 22, 26) du corps humain ou animal, la partie (22, 26) du corps étant conduite à travers ladite ouverture d'accès au corps (20) et placée à l'intérieur de ladite chambre interne (22), ledit sac enveloppant (18) présentant une paroi construite dans un matériau mince, flexible, transparent ; et
des moyens d'étanchéité (32) appliqués sur ladite paroi au niveau de ladite ouverture d'accès au corps (20) adaptés pour former un joint sensiblement étanche à l'air entre ledit sac enveloppant (18) et la partie du corps (14, 22, 26), n'autorisant que de lentes fuites dudit gaz depuis ladite chambre interne (22) ;
**caractérisée en ce que**
ladite paroi dudit sac (18) autour de ladite ouverture d'accès au corps (20) comporte une série de plis préformés (28), ladite ouverture d'accès (20) présentant une dimension d'ouverture initiale, lesdits plis préformés (28) étant formés par une série de lignes de pliage (30) parallèles et espacées, formées à l'intérieur de ladite paroi, lesdits plis préformés (28) permettant l'expansion de ladite dimension d'ouverture initiale de ladite ouverture d'accès (20), et permettant la contraction de ladite dimension d'ouverture initiale de ladite ouverture d'accès (20), lesdits plis préformés (28) facilitant le fronçage de ladite paroi au niveau de ladite ouverture d'accès pour former un ajustement serré avec la partie (14, 22, 26) du corps humain ou animal, lesdits plis préformés (28), lorsqu'ils sont plissés ensemble, permettant à des changements localisés de la forme du sac d'augmenter le volume d'oxygène au-dessus de la zone de la plaie.

2. Chambre selon la revendication 1, dans laquelle ladite paroi présente au moins un connecteur de drain (48), ledit connecteur de drain (48) étant adapté pour se connecter à un tube de drain (52) pour évacuer un liquide accumulé de l'intérieur de ladite chambre interne (22).

3. Chambre selon la revendication 1 ou 2, dans laquelle au moins une ouverture d'accès à la main (54) est formée à l'intérieur de ladite paroi, ladite ouverture d'accès à la main (54) comportant un élément de gant tubulaire (56), ledit élément de gant tubulaire (56) étant formé d'un matériau mince, flexible, étanche à l'air, ledit élément de gant tubulaire (56) étant mobile entre une position extérieure et une position intérieure, ladite position extérieure plaçant ledit élément de gant tubulaire à l'extérieur de ladite paroi, ladite position intérieure plaçant ledit élément de gant tubulaire (56) à l'intérieur de ladite chambre interne (22), la main d'une personne devant se connecter au dit élément de gant tubulaire (56) et déplacer ledit élément de gant tubulaire (56) à l'intérieur de ladite chambre interne (22), permettant à la main de la personne d'exécuter des fonctions sur ladite partie du corps (14, 24, 26) à l'intérieur de ladite chambre interne (22) tout en maintenant un environnement de gaz pressurisé dudit gaz à l'intérieur de ladite chambre interne (22).

4. Chambre selon l'une quelconque des revendications précédentes, dans laquelle :
ladite paroi présente au moins un connecteur d'alimentation (36, 38, 46) adapté pour être connecté à un tube d'alimentation en gaz (34) pour amener ledit gaz, ledit connecteur d'alimentation (36, 38, 46) présentant une tige tubulaire mâle (36) montée sur une bride élargie (46, 38), ladite bride élargie étant fixée rigidement à ladite paroi, ladite bride élargie (46, 38) empêchant une pliure dudit tube d'alimentation en gaz (34), garantissant une amenée continue de gaz à travers le tube d'alimentation en gaz (34) à l'intérieur de ladite chambre interne (22).

5. Chambre selon l'une quelconque des revendications précédentes, dans laquelle :
un capteur de pression (66) est connecté au dit sac enveloppant (18), ledit capteur de pression (66) étant capable d'indiquer le niveau de pression dudit gaz dans le sac enveloppant (18) à un observateur pendant le fonctionnement de ladite chambre à oxygène hyperbare (16).

6. Chambre selon la revendication 1, dans laquelle il est prévu un tube d'alimentation en gaz (34) et le sac (18) présente une extension à angle droit (58) en un coin du sac (18), le tube d'alimentation en gaz (34) s'étendant à travers un trou (60) dans l'extension à angle droit (58) sur une distance d'environ 203 mm (8 pouces) à l'intérieur de la chambre interne (22) du sac (18), réduisant de ce fait la possibilité de pliure du tube d'alimentation en gaz (34).
